# FASCICULE DE BREVET EUROPEEN

(11) **EP 1 431 030 B1**
(45) Date de publication et mention de la délivrance du brevet: **12.07.2006**
(21) Numéro de dépôt: 03293061.2
(22) Date de dépôt: 08.12.2003
(51) Int. Cl.: B32B 37/00, C12M 1/00

(54) **Recipient thermoforme pour la culture de cellules**
Thermogeformtes Zellkulturgefäss
Thermo-formed cell culture vessel

(30) Priorité: 20.12.2002 FR 0216439
(43) Date de publication de la demande: 23.06.2004
(73) Titulaire: MACO PHARMA, 59420 Mouvaux (FR)
(72) Inventeur: Heron, Antoine, 59250 Halluin (FR)
(74) Mandataire: Derambure, Christian

(56) Documents cités:
- EP-A- 0 890 636
- FR-A- 2 741 357
- US-A- 5 264 344
- US-A- 5 795 775
- US-B1- 6 245 555

## Description

L'invention concerne un récipient destiné à la culture de cellules, un système comprenant au moins deux éléments reliés entre eux en circuit clos au moyen d'une tubulure, dont un tel récipient, ainsi que l'utilisation d'un tel récipient, ou d'un tel système, pour la culture de cellules.

Le récipient selon l'invention est tout particulièrement destiné à la culture de cellules par adhérence à la surface intérieure du récipient. Bien entendu, le récipient peut également servir à la culture de cellules en suspension dans un milieu contenu dans le récipient.

L'avènement des cultures in vitro de cellules directement transplantables chez l'homme est à l'origine du développement de différents types de récipients pour le conditionnement desdites cultures. Utilisés dans un cadre médical, pour la préparation de produits de thérapie cellulaire et génique, ces récipients doivent présenter des garanties en matière de confinement des cellules et de prévention des risques de contamination et des erreurs techniques de manipulation. Lesdits récipients se doivent donc de respecter les règles strictes de bonne pratique des produits transfusables pour le conditionnement clos et le transfert des cellules.

De fait, l'utilisation de poches souples s'est accrue pour cultiver des cellules exploitées en thérapeutique humaine, notamment dans le cadre du développement des protocoles cliniques d'expansion ex vivo des cellules souches hématopoïétiques issues de prélèvement de moelle osseuse, de sang périphérique et de sang de cordon.

Les poches souples respectent les règles de bonnes pratiques précitées, cependant, elles présentent des inconvénients pour la culture de cellules.

Tout d'abord, la souplesse de ces poches ne permet pas de les empiler convenablement au sein d'un incubateur.

Ensuite, la flexibilité d'une poche définit une surface de culture flexible et par conséquent déformable en fonction du remplissage et des manipulations. Cette déformabilité engendre des zones de sédimentation et une répartition hétérogène des cellules sur la surface de culture disponible.

En outre, la surface de culture disponible est limitée par la taille de la poche. Pour satisfaire certaines applications nécessitant de grandes surfaces de culture, l'augmentation de la taille d'une poche ou la multiplicité de poches de petite taille accroît considérablement les difficultés de manipulation.

Enfin, les matériaux perméables aux gaz conventionnellement utilisés pour les poches souples de culture cellulaire, de type polyéthylène, polypropylène, polymères fluorés et éthylène-acétate de vinyle (EVA) ne permettent pas la culture de cellules adhérentes, mais uniquement la culture de cellules en suspension dans le milieu, ce qui limite considérablement les applications possibles. En effet, la majorité des cellules d'intérêt sont des cellules dont la culture s'effectue par adhérence.

Par ailleurs, on connaît du document US 6 297 046 une poche souple destinée à la culture de cellules, notamment par adhérence. De façon essentielle, la poche est formée par l'association de deux feuilles elles-mêmes composées d'un complexe de deux films dont l'un définit une surface interne de nature adhérente pour les cellules. La moindre flexibilité des films polymères de nature adhérente impose l'utilisation de films très minces dans la confection d'une poche souple d'où la nécessité de les complexer. En outre, l'utilisation de feuilles complexes limite la transparence de la poche et donc la possibilité d'observer le développement cellulaire au microscope. En plus des difficultés de réalisation de la poche décrite dans ce document, cette poche ne vise à satisfaire que le problème d'adhésion des cellules, alors que l'utilisateur attend une réponse globale à l'ensemble des problèmes mentionnés ci-dessus.

L'invention a notamment pour but de résoudre l'ensemble des inconvénients cités, en fournissant un récipient respectant les règles de bonnes pratiques mentionnées ci-dessus tout en permettant la culture de cellules par adhérence, ledit récipient étant empilable, et proposant des surfaces de culture homogènes et accrues pour un faible encombrement du récipient.

A cet effet, et selon un premier aspect, l'invention concerne un récipient destiné à la culture de cellules, comprenant une première et une deuxième feuilles perméables au gaz solidarisées l'une à l'autre au voisinage de leur périphérie de sorte à former un volume intérieur destiné à recevoir les cellules, et au moins une voie d'accès agencée pour permettre l'introduction et/ou la récupération des cellules.

Selon l'invention, chacune desdites feuilles comprend au moins une couche réalisée en une matière polymérique qui est apte à permettre l'adhérence des cellules, et au moins l'une des deux feuilles est thermoformée.

Le fait de réaliser un récipient à partir d'au moins une feuille de matière polymérique adhérente par la technique de thermoformage confère au récipient des caractéristiques particulièrement intéressantes, parmi lesquelles :
- le récipient présente une géométrie définie, ce qui permet d'empiler convenablement plusieurs récipients au sein d'un incubateur ;
- le récipient présente une certaine rigidité, ce qui permet d'éviter de créer des zones de sédimentation préférentielle et une hétérogénéité de répartition des cellules sur la surface de culture disponible.

Dans une réalisation particulière, chaque feuille est formée essentiellement de matière polymérique apte à permettre l'adhérence des cellules. La technique de thermoformage permet en effet de s'affranchir de l'emploi de feuilles complexes.

Selon une variante de l'invention, au moins l'une des feuilles thermoformées présente des reliefs qui sont disposés dans le volume intérieur du récipient.

La technique de thermoformage permet en effet la réalisation de reliefs disposés dans le volume intérieur du récipient. Ainsi, l'on peut considérablement augmenter la surface de culture disponible sans augmenter l'encombrement du récipient et sans augmenter le volume de milieu de culture consommé.

Ces reliefs peuvent former des motifs répétés ou irréguliers, continus ou discrets.

Selon une réalisation possible, les feuilles sont solidarisées l'une à l'autre par soudage au voisinage de leur périphérie.

Par exemple, au moins une première feuille est thermoformée de sorte à présenter, en coupe transversale, la forme générale d'un rectangle avec des angles arrondis comprenant un fond sensiblement plan, une paroi latérale et une paroi périphérique formant un rebord.

La deuxième feuille, formant la paroi supérieure du récipient, peut être :
- soit solidarisée au rebord de la première feuille de sorte à être sensiblement plane ;
- soit thermoformée de sorte à présenter une géométrie analogue à celle de la première feuille, lesdites feuilles étant solidarisées en regard l'une de l'autre par leurs rebords.

Le récipient peut comprendre au moins une voie d'accès communiquant avec le volume intérieur du récipient depuis une paroi d'une feuille thermoformée, par exemple une paroi latérale, périphérique ou supérieure.

Selon un deuxième aspect, l'invention concerne un système comprenant au moins deux éléments reliés entre eux en circuit clos au moyen d'une tubulure, l'un au moins desdits éléments étant un récipient tel que précédemment décrit, la tubulure étant connectée à une première extrémité à la voie d'accès du récipient et à une deuxième extrémité à une voie d'accès d'un autre élément du système, de sorte à permettre le passage des cellules ou des fluides entre les éléments du système.

Enfin, selon un troisième aspect, l'invention concerne l'utilisation d'un tel récipient ou d'un tel système pour la culture de cellules adhérentes ou en suspension dans le milieu.

Les autres caractéristiques de l'invention résultent de la description qui suit de modes de réalisation, effectuée en référence aux figures annexées dans lesquelles :
- la figure 1 est une représentation schématique en coupe transversale d'un récipient formé d'une feuille thermoformée et d'une feuille, le récipient étant muni de deux voies d'accès ;
- les figures 2a à 2c sont des représentations schématiques en coupe transversale de récipients formés d'une feuille thermoformée présentant des reliefs sur sa face intérieure et d'une feuille, les récipients étant munis d'une ou deux voies d'accès ;
- les figures 3a à 3f sont des représentations schématiques en coupe transversale de récipients formés de deux feuilles thermoformées, présentant des reliefs ou non, les récipients étant munis d'une ou deux voies d'accès ;
- la figure 4a est une représentation schématique en coupe transversale d'un récipient formé d'une feuille thermoformée et d'une feuille, le récipient étant muni, sur la paroi périphérique de la feuille thermoformée, d'une voie d'accès orientée verticalement vers le bas ;
- la figure 4b est une représentation schématique en coupe transversale d'un récipient formé de deux feuilles thermoformées, le récipient étant muni, sur la paroi périphérique d'une des feuilles thermoformées, d'une voie d'accès orientée verticalement vers le haut ;
- la figure 4c représente de façon schématique la structure d'une voie d'accès ;
- la figure 4d représente de façon schématique la structure d'une voie d'accès associée à un renfort interne ;
- les figures 5a à 5d représentent différentes structures que peuvent prendre les reliefs de la ou des feuilles thermoformées.

Un récipient 1 selon l'invention comprend deux feuilles 2, 3 respectivement inférieure et supérieure, solidarisées l'une à l'autre au voisinage de leur périphérie.

Selon une réalisation possible, les feuilles 2, 3 sont soudées. Toutefois, les feuilles 2, 3 peuvent également être solidarisées par une méthode différente, notamment par collage.

Le récipient 1 définit ainsi un volume intérieur destiné à recevoir des cellules et un milieu de culture.

Les deux feuilles 2, 3, sont perméables aux gaz, notamment à l'oxygène, et réalisées en une matière polymérique biocompatible, transparente, et sur laquelle les cellules peuvent adhérer. De la sorte, le récipient 1 permet un très bon développement des cellules, et sa transparence offre la possibilité de suivre la production cellulaire en microscopie optique.

A titre d'exemple de matière polymérique utilisée pour les feuilles 2, 3, on peut citer le polyester notamment sous forme APET ou PETG, le polycarbonate ou le polystyrène.

Par ailleurs, le potentiel d'adhérence cellulaire des polymères utilisés pour les feuilles 2, 3 peut être facilement accru par différents traitements de surface connus, notamment le greffage chimique, ou un traitement par gaz activants. De façon préférentielle, un traitement de surface de type plasma (plasma / oxygène ou plasma / air) est réalisé spécifiquement sur la surface destinée à la culture avant la fermeture du récipient 1.

Selon l'invention, au moins la feuille inférieure 2 est thermoformée. Le récipient 1 présente, en coupe transversale, la forme générale d'un rectangle avec des angles arrondis, comprenant une paroi formant le fond 4 du récipient 1 entourée par une paroi latérale 5, prolongé latéralement par une paroi périphérique 6 formant un rebord destiné à être solidarisé à la feuille supérieure 3. La présence de ces angles arrondis garantit une récupération optimale des produits cellulaires après la culture.

Les feuilles 2, 3 peuvent avoir des épaisseurs variables et des niveaux variables de perméabilité aux gaz. Par exemple, l'épaisseur d'une feuille est comprise entre 100 et 500 µm. Cette faible épaisseur permet d'obtenir une perméabilité satisfaisante aux gaz, notamment à l'oxygène.

Le thermoformage conduit à une réduction de l'épaisseur de la feuille. Il est alors possible de jouer avec ce phénomène pour faire varier la perméabilité aux gaz du récipient 1.

De plus, la réalisation de récipients de profondeurs différentes permet également de jouer sur l'oxygénation du milieu. En effet, certaines cellules, notamment les cellules souches hématopoiétiques, poussent préférablement dans un milieu avec peu d'oxygène : dans ce cas, il est judicieux d'utiliser un récipient peu profond rempli exclusivement de milieu de culture, de façon à limiter les échanges de gaz. Par contre, d'autres types de cellules, comme les hépatocytes, sont très consommatrices d'oxygène : un récipient plus profond rempli partiellement de milieu de culture permet alors d'obtenir une interface avec un volume d'air contenu dans le récipient, et par conséquent de favoriser les échanges gazeux et notamment l'apport en oxygène. Dans ce cadre, l'utilisation de la technique de thermoformage permet de réaliser des récipients de profondeurs différentes très facilement, sans qu'il soit nécessaire de créer un moule pour chaque type de récipient à réaliser. Une modification du calage du moule suffit à modifier le volume intérieur du récipient.

Selon un deuxième aspect, l'invention concerne un système comprenant plusieurs éléments, dont au moins un récipient selon l'invention, associés entre eux de manière à former un circuit clos. Un tel système peut notamment comporter des éléments aptes à prélever, transférer, alimenter, concentrer, filtrer, inactiver ou laver les produits cellulaires. Par exemple, ces éléments peuvent être composés de poches souples de conditionnement des milieux et réactifs de culture cellulaire et de poches souples de transfert et centrifugation des produits cellulaires. Dans ce cadre, la notion de système clos intégrant au moins un récipient selon l'invention, a pour but de sécuriser l'ensemble des manipulations effectuées pour la production de cellules par culture.

Il peut être judicieux de limiter la perméabilité aux gaz du récipient 1 lorsque celui-ci s'intègre dans un système capable de piloter les apports de gaz aux cellules. On peut facilement limiter cette perméabilité, notamment en utilisant des feuilles plus épaisses.

Un pilotage des fluides au sein du système définit un bioréacteur de culture cellulaire. Dans ce cadre, le système est en mesure d'alimenter en continu ou de façon séquentielle les cellules en culture par la mise en circulation des milieux et réactifs. Le système peut être également doté d'un ensemble de moyens de contrôle et de régulation. Ces moyens permettent notamment d'appliquer les valeurs de temps, de température, de pH du milieu et de la teneur en gaz, sélectionnées pour une application donnée. Un système pilotable ou bioréacteur trouve un intérêt tout particulièrement dans la mise en oeuvre de culture cellulaire à long terme ; on peut citer par exemple la production de cellules souches mésenchymateuses extraites de la moelle osseuse ou la production de cellules souches hématopoïétiques en coculture sur des cellules stromales adhérentes.

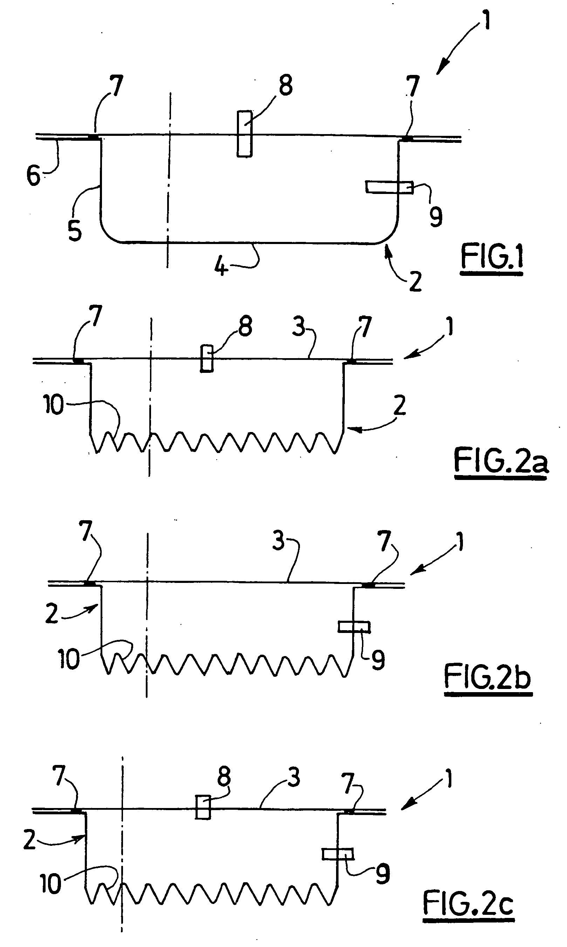

Selon un premier mode de réalisation de l'invention, représenté sur les figures 1 et 2a à 2c, la feuille supérieure 3 n'est pas thermoformée.

Les feuilles 2, 3 sont solidarisées l'une à l'autre au niveau d'une zone de soudage 7 située par exemple sur la paroi périphérique 6 de la feuille inférieure 2, à proximité de la paroi latérale 5. La feuille supérieure 3 est disposée de sorte à être sensiblement plane.

Sur la figure 1, la feuille inférieure 2 ne présente pas de reliefs. Le fond 4, en particulier, étant sensiblement plan et lisse, offre une surface homogène pour la répartition et la culture des cellules.

Par ailleurs, le récipient 1 comporte deux orifices agencés pour permettre l'introduction et/ou la récupération des cellules, au moyen de voies d'accès coopérant avec lesdits orifices. Une première voie d'accès 8 communique avec l'intérieur du récipient 1 depuis la paroi supérieure du récipient 1 formée par la feuille supérieure 3, et une seconde voie d'accès 9 communique avec l'intérieur du récipient 1 depuis la paroi latérale 5 de la feuille inférieure 2 thermoformée.

Sur les figures 2a à 2c, la feuille inférieure 2 présente des reliefs 10 sur sa face intérieure au récipient 1, essentiellement sur le fond 4, le fond conservant une forme globalement plane et homogène.

Les reliefs 10 permettent de résoudre le problème des grandes surfaces nécessaires à la culture de certaines cellules en système clos. En effet, la production de cellules humaines adhérentes (cellules mésenchymateuses, musculaires, neuronales...) se trouve limitée par la densité maximale des cellules par unité de surface au-delà de laquelle la prolifération cellulaire cesse. Le nombre minimal de cellules requises pour une greffe impose donc une surface minimale de culture cellulaire, celle-ci devant être le plus souvent supérieure au mètre carré.

L'utilisation de la technique de thermoformage permet de structurer la surface de culture cellulaire et d'augmenter considérablement la surface de culture disponible pour un récipient 1 ayant le même encombrement.

L'on comprend ainsi l'intérêt de former des reliefs 10 sur le fond 4 et à l'intérieur du récipient 1, tandis que la réalisation de tels reliefs 10 sur les parois latérales 5 n'est pas indispensable, dans la mesure où les cellules vont se déposer, par gravité, sur le fond 4.

Dans un exemple de réalisation particulier, les feuilles 2, 3 thermoformées et structurées en relief sont munies de zones planes pour faciliter l'observation des cellules au microscope, et l'apposition de voies d'accès le cas échéant.

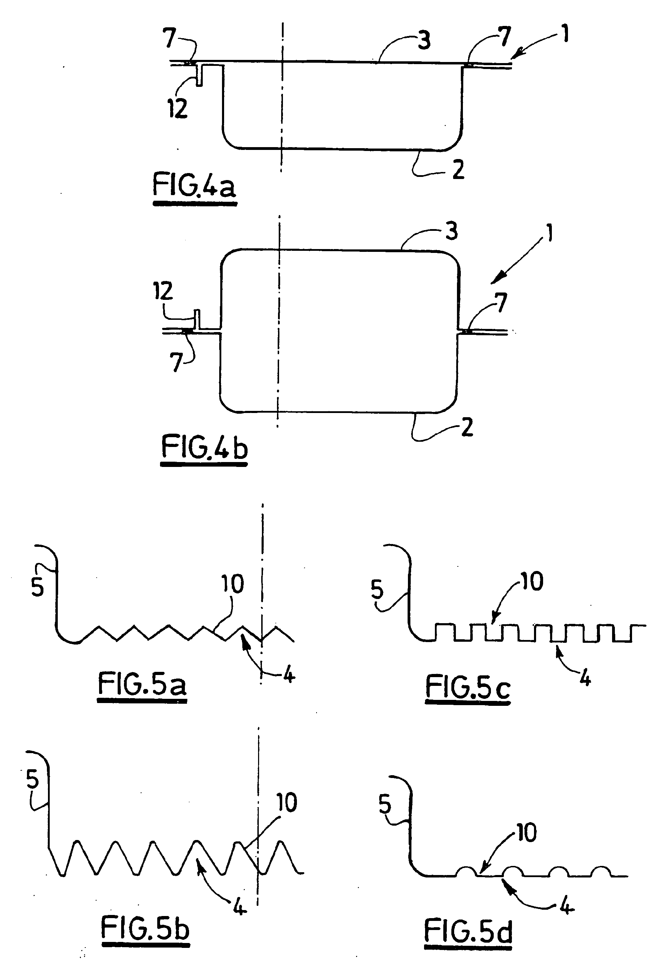

Les reliefs 10 peuvent prendre différentes formes, comme il sera décrit plus loin en référence aux figures 5a à 5d.

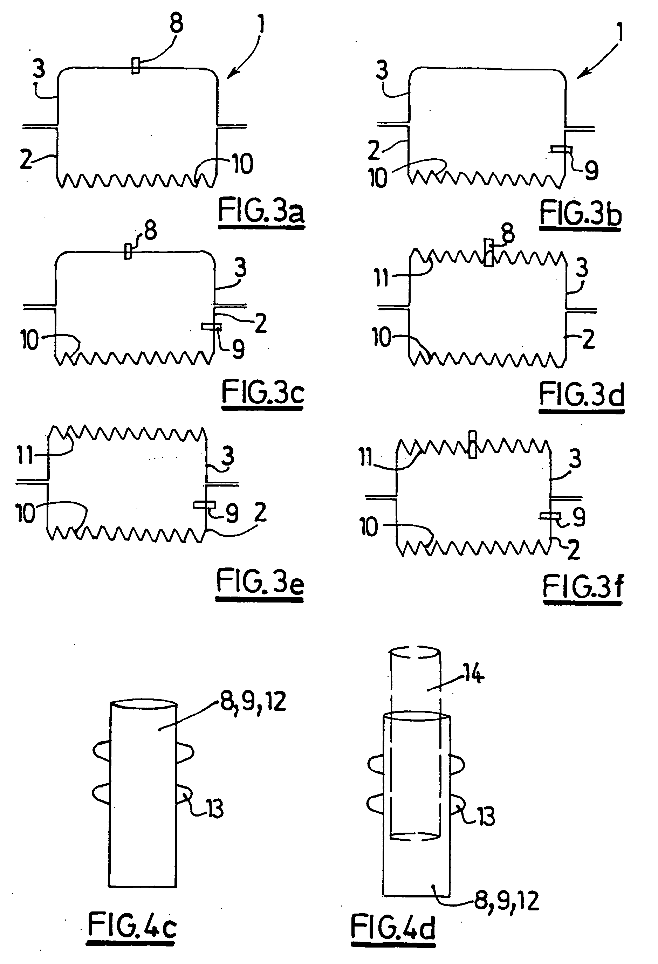

Le récipient 1 représenté sur les figures 3a à 3f est formé d'une feuille inférieure 2 thermoformée, et d'une feuille supérieure 3 également thermoformée, par exemple de géométrie analogue ou sensiblement identique à celle de la feuille inférieure 2. Les feuilles 2, 3 sont solidarisées en regard l'une de l'autre.

La feuille inférieure 2 présente des reliefs 10 sur sa face intérieure au récipient 1, la feuille supérieure 3 pouvant également présenter de tels reliefs 11 sur sa face intérieure au récipient 1 (figures 3d, 3e, 3f) ou, au contraire, présenter une surface sensiblement plane et lisse (figures 3a, 3b, 3c). Lorsque les deux feuilles 2, 3 présentent des reliefs 10, 11, le récipient 1 peut être posé soit sur la feuille inférieure 2, soit sur la feuille supérieure 3 pour la culture des cellules par adhérence ; la culture de cellules adhérentes peut alors être envisagée au contact des deux feuilles 2, 3 simultanément, ce qui double la capacité de production cellulaire déjà optimisée dudit récipient 1.

La figure 4a représente un récipient 1 formé d'une feuille inférieure 2 thermoformée ne présentant pas de reliefs et d'une feuille supérieure 3, solidarisées l'une à l'autre au niveau d'une zone de soudage 7. La feuille supérieure 3 est disposée de sorte à être sensiblement plane.

La figure 4b est similaire à la figure 4a, la feuille supérieure 3 étant néanmoins thermoformée, et ne présentant pas de reliefs.

Différentes réalisations sont envisageables en ce qui concerne les voies d'accès. Le récipient 1 peut ainsi comporter, soit une voie d'accès 8 communiquant avec l'intérieur du récipient 1 depuis la paroi supérieure du récipient 1 formée par la feuille supérieure 3 (figures 2a, 3a, 3d), soit une voie d'accès 9 communiquant avec l'intérieur du récipient 1 depuis la paroi latérale 5 de la feuille inférieure 2 (figures 2b, 3b, 3e), soit les deux voies d'accès 8, 9 (figures 1, 2c, 3c, 3f).

Le récipient 1 présente également une voie d'accès 12 associée à la paroi périphérique 6 de la feuille inférieure 2 (figure 4a) ou à la paroi périphérique de la feuille supérieure 3 (figure 4b).

Les voies d'accès 8, 9 sont soudées aux feuilles 2, 3, mais peuvent également être solidarisées auxdites feuilles 2, 3 notamment par collage.

Toutefois, ces voies d'accès périphériques 8, 9, 12 peuvent être réalisées simplement par la technique du thermoformage. Dans un premier temps, une protubérance est créée sur la paroi de la feuille 2, 3. Dans un deuxième temps, les protubérances sont perforées afin de créer les voies d'accès 8, 9, 12 et permettre notamment de raccorder une tubulure.

Selon une réalisation possible, représentée sur les figures 1, 2b, 2c, 3b, 3c, 3e et 3f, la protubérance est créée sur la paroi latérale définit par le thermoformage de la feuille inférieure 2. L'utilisation de moules de thermoformage munis de parties amovibles permet de retirer la pièce ainsi formée.

Selon une autre réalisation possible, représentée sur les figures 4a et 4b, la protubérance est créée sur la feuille de façon à être orientée perpendiculairement à la zone périphérique de la feuille, cette orientation permettant de faciliter la production puisque l'utilisation de moules munis de parties amovibles n'est alors plus nécessaire.

La réalisation du récipient selon l'invention et de ses voies d'accès 8, 9, 12 permet de s'affranchir de l'insertion desdites voies d'accès entre les deux feuilles constituant ledit récipient, comme tel est le cas dans la fabrication d'une poche souple. La zone de solidarisation des deux feuilles reste donc entièrement plane, cela constitue un élément important pour souder des matériaux de nature adhérente pour les cellules, en particulier des feuilles non complexées à base de films en polyester, en polycarbonate ou en polystyrène.

En outre, l'intégration des voies d'accès 8, 9, 12 dans au moins une feuille thermoformée permet d'éliminer les risques de fuite qui peuvent exister au niveau de la zone d'insertion desdites voies entre les feuilles des récipients de l'art antérieur.

La réalisation du récipient 1 n'exclut pas la possibilité d'apposer les voies d'accès, notamment sous la forme de tubes, entre les deux feuilles 2 et 3 (non représentées). Compte-tenu de l'emploi préférentiel de films polymères de moindre flexibilité, il conviendra d'y préformer les zones d'emplacement de ces tubes, une condition satisfaite également par le thermoformage des feuilles 2 et 3.

Comme représenté sur la figure 4c, il est envisageable d'ajouter sur les voies d'accès 8, 9, 12 des cannelures 13 afin d'améliorer l'étanchéité avec une tubulure. On obtient ainsi des embouts cannelés (il est également envisageable de structurer des embouts coniques à olives, afin d'atteindre le même résultat).

Comme représenté sur la figure 4d, il est également envisageable d'ajouter à l'intérieur des voies d'accès 8, 9, 12 un renfort 14 interne permettant également d'obtenir un raccord parfaitement étanche avec une tubulure.

En effet, les feuilles utilisées sont généralement de faible épaisseur pour assurer un niveau minimum de perméabilité aux gaz, notamment à l'oxygène, et le processus de thermoformage réduit encore cette épaisseur. Il convient donc de renforcer les voies d'accès 8, 9, 12 pour garantir leur solidité ainsi que leur étanchéité une fois raccordées à une tubulure.

On se réfère maintenant aux figures 5a à 5d, qui illustrent différentes formes possibles des reliefs 10, 11.

Les reliefs 10, 11 peuvent se présenter notamment sous la forme de plis, d'ondulations, de créneaux, de picots, respectivement représentés sur les figures 5a, b, c et d. Les reliefs 10, 11 peuvent former des motifs répétés ou être irréguliers. Les reliefs 10, 11 peuvent s'étendre sur une partie ou sur la totalité du fond 4 du récipient 1.

Afin d'obtenir des augmentations de surface de culture suffisantes, ces reliefs 10, 11 ne sont pas réalisés à l'échelle micrométrique ou nanométrique, mais au moins à l'échelle millimétrique.

Comme indiqué précédemment, la réalisation de reliefs 10, 11 sur la face des feuilles 2, 3 intérieure au récipient 1 par thermoformage permet d'augmenter la surface de culture pour les cellules adhérentes.

Ces reliefs peuvent également être un moyen de retenir les cellules non-adhérentes sur la feuille 2, 3 lors de l'application d'une circulation de milieu, dans le cadre d'une culture cellulaire sous perfusion continue de milieu au sein du récipient 1.

Sur ce point, un relief, ayant une forme en créneaux, représentée sur la figure 5c, disposés dans le volume intérieur du récipient 1 serait particulièrement adapté à la culture de cellules non adhérentes sous perfusion de milieu.

La demanderesse a développé la technique de thermoformage pour la réalisation du récipient selon l'invention. Le récipient selon l'invention innove par l'apport d'une réponse globale à l'ensemble de critères limitants dans l'emploi d'une poche souple de culture cellulaire. En outre, cette technologie de transformation des matières plastiques est particulièrement bien adaptée à la réalisation d'une gamme de récipients de culture cellulaire dont les caractéristiques dimensionnelles et structurales peuvent être aisément adaptées en fonction des types cellulaires et de leurs applications. Bien que particulièrement adapté à la préparation de cellules à des fins thérapeutiques, le récipient selon l'invention pourra trouver d'autres applications biotechnologiques exploitant les cultures de cellules procaryotes comme eucaryotes.

## Revendications

1. Récipient (1) destiné à la culture de cellules, comprenant une première (2) et une deuxième (3) feuilles perméables aux gaz solidarisées l'une à l'autre au voisinage de leur périphérie (6) de sorte à former un volume intérieur destiné à recevoir les cellules, et au moins une voie d'accès agencée pour permettre l'introduction et/ou la récupération des cellules, chacune desdites feuilles (2, 3) comprenant au moins une couche réalisée en une matière polymérique qui est apte à permettre l'adhérence des cellules, **caractérisé en ce qu'**au moins l'une des deux feuilles (2, 3) est thermoformée.

2. Récipient selon la revendication 1, **caractérisé en ce que** chaque feuille (2, 3) est formée essentiellement de matière polymérique apte à permettre l'adhérence des cellules.

3. Récipient selon la revendication 1 ou 2, **caractérisé en ce que** les feuilles (2, 3) sont solidarisées l'une à l'autre par soudage au voisinage de leur périphérie.

4. Récipient selon l'une quelconque des revendications 1 à 3, **caractérisé en ce qu'**au moins une première feuille (2) est thermoformée de sorte à présenter, en coupe transversale, la forme générale d'un rectangle comprenant un fond (4) sensiblement plan, une paroi latérale (5) et une paroi périphérique (6) formant un rebord.

5. Récipient selon la revendication 4, **caractérisé en ce que** la deuxième feuille (3) est solidarisée au rebord de la première feuille (2) de sorte à être sensiblement plane, ladite deuxième feuille (3) formant la paroi supérieure du récipient (1).

6. Récipient selon la revendication 4, **caractérisé en ce que** la deuxième feuille (3), formant la paroi supérieure du récipient (1), est thermoformée de sorte à présenter une géométrie analogue à celle de la première feuille (2), lesdites feuilles (2, 3) étant solidarisées en regard l'une de l'autre par leurs rebords.

7. Récipient selon l'une quelconque des revendications 1 à 6, **caractérisé en ce qu'**au moins une voie d'accès (9) communique avec le volume intérieur du récipient (1) depuis une paroi d'une feuille thermoformée (2, 3).

8. Récipient selon l'une quelconque des revendications 1 à 7, **caractérisé en ce qu'**au moins l'une des feuilles thermoformées (2, 3) présente des reliefs (10, 11) qui sont disposés dans le volume intérieur du récipient (1).

9. Récipient selon la revendication 8, **caractérisé en ce que** les reliefs (10, 11) forment des motifs répétés ou irréguliers, continus ou discrets.

10. Système comprenant au moins deux éléments reliés entre eux en circuit clos au moyen d'une tubulure, l'un au moins desdits éléments étant un récipient (1) conforme à l'une quelconque des revendications 1 à 9, la tubulure étant connectée à une première extrémité à la voie d'accès du récipient (1) et à une deuxième extrémité à un autre élément du système, de sorte à permettre le passage des cellules et/ou des fluides entre les éléments du système.

11. Utilisation d'un récipient (1) selon l'une quelconque des revendications 1 à 9 ou d'un système selon la revendication 10 pour la culture de cellules adhérentes et/ou en suspension dans le milieu.

## Claims

1. A receptacle (1) intended for cell culture, comprising first (2) and second (3) sheets permeable to gases, fixed to each other close to their periphery (6) so as to form an internal volume intended to receive the cells, and at least one access channel arranged so as to allow the introduction and/or recovery of the cells, each of the said sheets (2, 3) comprising at least one layer produced from a polymeric material and which is suitable for allowing the adhesion of the cells, **characterised in that** at least one of the two sheets (2, 3) is thermoformed.

2. A receptacle according to claim 1, **characterised in that** each sheet (2, 3) is formed essentially from polymeric material suitable for allowing adhesion of the cells.

3. A receptacle according to claim 1 or 2, **characterised in that** the sheets (2, 3) are fixed to each other by welding close to their periphery.

4. A receptacle according to any one of claims 1 to 3, **characterised in that** at least a first sheet (2) is thermoformed so as to have, in transverse section, the general shape of a rectangle comprising a substantially flat bottom (4), a lateral wall (5) and a peripheral wall (6) forming a rim.

5. A receptacle according to claim 4, **characterised in that** the second sheet (3) is fixed to the rim of the first sheet (2) so as to be substantially flat, the said second sheet (3) forming the top wall of the receptacle (1).

6. A receptacle according to claim 4, **characterised in that** the second sheet (3) forming the top wall of the receptacle (1) is thermoformed so as to have a geometry similar to that of the first sheet (2), the said sheets (2, 3) being fixed opposite each other by their rims.

7. A receptacle according to any one of claims 1 to 6, **characterised in that** at least one access channel (9) communicates with the internal volume of the receptacle (1) from one wall of a thermoformed sheet (2, 3).

8. A receptacle according to any one of claims 1 to 7, **characterised in that** at least one of the thermoformed sheets (2, 3) has reliefs (10, 11) which are disposed in the internal volume of the receptacle.

9. A receptacle according to claim 8, **characterised in that** the reliefs (10, 11) form repeated or irregular patterns, continuous or discrete.

10. A system comprising at least two elements connected together in closed circuit by means of a tube, at least one of the said elements being a receptacle (1) according to any one of claims 1 to 9, the tube being connected at a first end to the access channel of the receptacle (1) and at a second end to the other element of the system, so as to allow the passage of the cells and/or of the fluids between the elements of the system.

11. Use of a receptacle (1) according to any one of claims 1 to 9 or of a system according to claim 10 for the culture of cells adhering and/or in suspension in the medium.

## Patentansprüche

1. Behälter (1) für die Zellenkultur mit einer ersten (2) und einer zweiten (3) gasdurchlässigen Folie, die an ihrem Umfang (6) fest miteinander verbunden sind, um ein Innenvolumen für die Aufnahme der Zellen zu bilden, und mit mindestens einem Zugangskanal für die Einführung und/oder die Entnahme der Zellen, wobei jede der besagten Folien (2, 3) mindestens eine aus einem Polymermaterial bestehende Schicht aufweist, die das Anhaften der Zellen ermöglichen kann, **dadurch gekennzeichnet, dass** zumindest eine der Folien (2, 3) wärmegeformt ist.

2. Behälter nach Anspruch 1, **dadurch gekennzeichnet, dass** jede Folie (2, 3) im wesentlichen aus einem Polymermaterial gebildet ist, das das Anhaften der Zellen ermöglichen kann.

3. Behälter nach Anspruch 1 oder 2, **dadurch gekennzeichnet, dass** die Folien (2, 3) durch Verschweißen in der Nähe ihres Umfangs fest miteinander verbunden sind.

4. Behälter nach einem der Ansprüche 1 bis 3, **dadurch gekennzeichnet, dass** zumindest eine erste Folie (2) wärmegeformt ist, so dass sie im Querschnitt eine im allgemeinen rechteckige Form aufweist, mit einem etwa ebenen Boden (4), einer Seitenwand (5) und einer einen Rand bildenden Umfangswand (6).

5. Behälter nach Anspruch 4, **dadurch gekennzeichnet, dass** die zweite Folie (3) fest mit dem Rand der ersten Folie (2) verbunden ist, so dass sie etwa eben ist, wobei die besagte zweite Folie (3) die obere Wand des Behälters (1) bildet.

6. Behälter nach Anspruch 4, **dadurch gekennzeichnet, dass** die zweite, die obere Wand des Behälters (1) bildende Folie (3) wärmegeformt ist, so dass sie eine ähnliche Geometrie aufweist wie diejenige der ersten Folie (2), wobei die besagten Folien (2, 3) sich gegenüberliegend an ihren Rändern fest miteinander verbunden sind.

7. Behälter nach einem der Ansprüche 1 bis 6, **dadurch gekennzeichnet, dass** zumindest ein Zugangskanal (9) von einer Wand einer wärmegeformten Folie (2, 3) aus mit dem Innenvolumen des Behälters (1) in Verbindung steht.

8. Behälter nach einem der Ansprüche 1 bis 7, **dadurch gekennzeichnet, dass** zumindest eine der wärmegeformten Folien (2, 3) Reliefs (10, 11) aufweist, die in dem Innenvolumen des Behälters (1) angeordnet sind.

9. Behälter nach Anspruch 8, **dadurch gekennzeichnet, dass** die Reliefs (10, 11) wiederholte oder unregelmäßige, durchgehende oder diskrete Muster bilden.

10. System mit mindestens zwei mittels einem Stutzen in geschlossenem Kreis miteinander verbundenen Elementen, wobei zumindest eines der besagten Elemente ein Behälter (1) nach einem der Ansprüche 1 bis 9 ist, wobei der Stutzen an einem ersten Ende an dem Zugangskanal des Behälters (1) und an einem zweiten Ende an einem anderen Element des Systems angeschlossen ist, um die Passage der Zellen und/oder der Flüssigkeiten zwischen den Elementen des Systems zu ermöglichen.

11. Verwendung eines Behälters (1) nach einem der Ansprüche 1 bis 9 oder eines Systems nach Anspruch 10 für die Kultur von anhaftenden und/oder in dem Milieu in Suspension befindlichen Zellen.
